# EUROPÄISCHE PATENTSCHRIFT

(11) **EP 3 143 185 B1**
(45) Veröffentlichungstag und Bekanntmachung des Hinweises auf die Patenterteilung: **03.10.2018**
(21) Anmeldenummer: 15723477.4
(22) Anmeldetag: 12.05.2015
(51) Int. Cl.: C25B 3/02, C25B 11/12, C07C 211/58, C07C 211/59, C07C 209/36, C07C 209/62, C07D 213/127, C07D 213/16

(54) **VERFAHREN ZUR ELEKTROCHEMISCHEN AMINIERUNG AN BOR-DOTIERTEN DIAMANTANODEN**
METHOD FOR THE ELECTROCHEMICAL AMINATION AT BORON DOPED DIAMOND ANODES
PROCÉDÉ D'AMINATION ÉLECTROCHIMIQUE SUR DES ANODES DE DIAMANT DOPÉES AU BORE

(30) Priorität: 15.05.2014 EP 14168447
(43) Veröffentlichungstag der Anmeldung: 22.03.2017
(73) Patentinhaber: Covestro Deutschland AG, 51373 Leverkusen (DE)
(72) Erfinder: RICHTER, Frank, 51373 Leverkusen (DE); HECKROTH, Heike, 51519 Odenthal (DE); HALPAAP, Reinhard, 51519 Odenthal (DE); WALDVOGEL, Siegfried R., 55435 Gau-Algesheim (DE); HEROLD, Sebastian, 63075 Offenbach am Main (DE); NEFZGER, Hartmut, 50259 Pulheim (DE)
(74) Vertreter: Levpat
(86) Internationale Anmeldenummer: PCT/EP2015/060438
(87) Internationale Veröffentlichungsnummer: WO 2015/173221

(56) Entgegenhaltungen:
- EP-A1- 1 036 861
- WO-A1-2010/000600
- TATSUYA MOROFUJI ET AL: "Electrochemical C-H Amination: Synthesis of Aromatic Primary Amines via N -Arylpyridinium Ions", JOURNAL OF THE AMERICAN CHEMICAL SOCIETY, Bd. 135, Nr. 13, 3. April 2013 (2013-04-03), Seiten 5000-5003, XP055137525, ISSN: 0002-7863, DOI: 10.1021/ja402083e
- HANS SIGRIST ET AL: "5-Isothiocyanato-1-naphthalene Azide and p-Azidophenylisothiocyanate. Synthesis and Application in Hydrophobic Heterobifunctional Photoactive Cross-Linking of Membrane Proteins", EUROPEAN JOURNAL OF BIOCHEMISTRY, Bd. 125, Nr. 1, 1. Juni 1982 (1982-06-01), Seiten 197-201, XP055137812, ISSN: 0014-2956, DOI: 10.1111/j.1432-1033.1982.tb06668.x
- ILUMINADA GALLARDO ET AL: "Electrochemical Synthesis of Nitroanilines", EUROPEAN JOURNAL OF ORGANIC CHEMISTRY, Bd. 2002, Nr. 2, 1. Januar 2002 (2002-01-01), Seiten 251-259, XP055137544, ISSN: 1434-193X, DOI: 10.1002/1099-0690(20021)2002:2<251::AID-EJ OC251>3.0.CO;2-A
- HUGO CRUZ ET AL: "Electrochemically promoted nucleophilic aromatic substitution in room temperature ionic liquids-an environmentally benign way to functionalize nitroaromatic compounds", GREEN CHEMISTRY, Bd. 13, Nr. 9, 20. Juli 2011 (2011-07-20), Seiten 2531-2542, XP055137536, ISSN: 1463-9262, DOI: 10.1039/c1gc15303j

## Beschreibung

Die gezielte und selektive Herstellung von definierten Isomeren ist bei der Zweitsubstitution am Aromaten häufig schwierig. Man erhält in der Regel Isomerengemische sowie mehr als zweifach substituierte Spezies. So ist die gezielte Synthese von in 1,5-Stellung disubstituierten Naphthalinderivaten bisher nicht möglich.

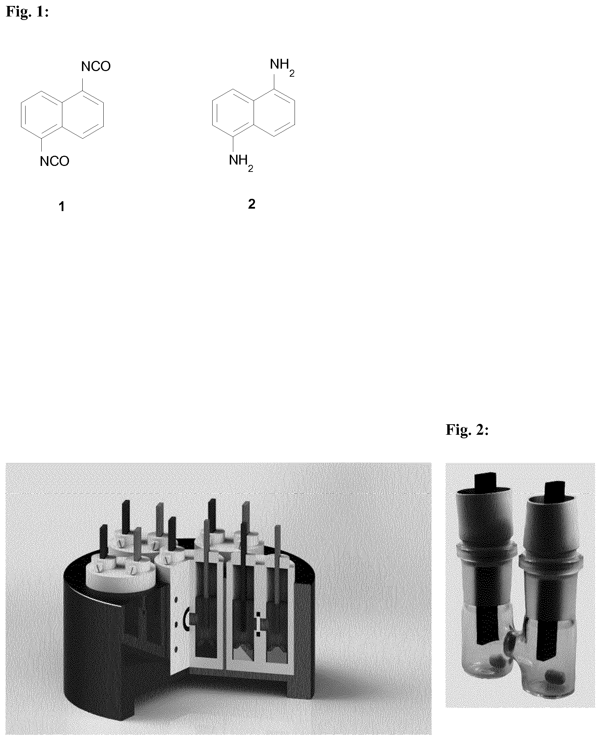

Insbesondere 1,5-Diaminonaphthalin (**2**) stellt eine bedeutende Zwischenstufe zur Herstellung von 1,5-Naphthalindiisocyanat (**1**) dar (Figur 1). Dieses aromatische Diisocyanat ist ein wichtiger Grundbaustein für hochbelastbare Polyurethane.

Die industrielle Synthese von 1,5-Naphthalindiisocyanat erfolgt ausgehend von Naphthalin (**3**), welches zu 1,5-Naphthalindisulfonsäure (**4**) umgesetzt wird. 1,5-Naphthalindisulfonsäure wird in einer Alkalischmelze zu 1,5-Dihydroxynaphthalin (**5**) umgesetzt, welches dann in einer Bucherer-Reaktion in das Diamin **2** überführt wird. Anschließend erfolgt die Phosgenierung zum 1,5-Naphthalindiisocyanat (**1**). Diese klassische Synthese von 1,5-Naphthalindiisocyanat ist in Schema 1 dargestellt.

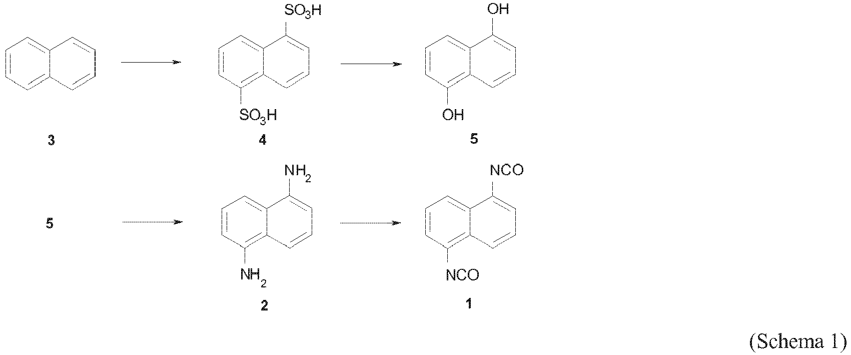

Gravierende Nachteile des hier beschriebenen Prozesses sind die Entstehung von IsomerenGemischen bei der Sulfonierung von Naphthalin. Neben der gewünschten 1,5-Verbindung fallen weitere Disulfonsäuren sowie Trisulfonsäuren an. Dies ist mit einem hohen Ausbeuteverlust bezogen auf das eingesetzte Naphthalin verbunden. Die drastischen und korrosiven Bedingungen bei der Reaktion zur Dihydroxyverbindung stellen weitere Makel des klassischen Prozesses dar. Die vielen Syntheseschritte sind mit zeitintensiven Aufarbeitungen der Zwischenprodukte verbunden. Zudem fallen durch die vielen Prozessstufen hohe energetische Kosten an. Knapper werdende Rohstoffe und die steigende Relevanz des Umweltschutzes erfordern daher neue Synthesestrategien zur Darstellung von 1,5-Diamoninaphthalin (**2**).

WO 2010/000600 A1 offenbart ein Verfahren zur elektrochemischen Direktaminierung von Kohlenwasserstoffen. EP 1 036 861 A1 offenbart diamantbeschichtete Elektroden.

Die Aufgabe der Erfindung ist es, einen umweltschonenden und zugleich kostengünstigen Zugang zu in 1,5-Stellung disubstituierten Naphthalinderivaten, insbesondere zu 1,5-Diaminonaphthalin aufzuzeigen. Eine kompetitive synthetische Darstellung von 1,5-Diaminonaphthalin, einer wichtigen Vorstufe für hochbelastbare Polyurethane, kann zu einem breiteren Einsatz dieser robusten und langlebigen Polymere führen.

Gegenstand der Erfindung ist ein Verfahren gemäß Anspruch 1.

Unter deaktivierten aromatischen Systemen sind substituierte Aromaten zu verstehen, deren Elektronendichte im Ring durch induktive und/oder mesomere Effekte der Substituenten verringert ist. Unter nicht-aktivierten aromatischen Systemen sind nicht-substituierte Aromaten zu verstehen. Weist ein substituierter Aromat sowohl Substituenten, die Elektronendichte im Ring durch induktive und/oder mesomere Effekte verringern, als auch Substituenten, die Elektronendichte im Ring durch induktive und/oder mesomere Effekte erhöhen, auf, so fällt ein solcher Aromat unter die Definition eines deaktivierten aromatischen Systems, wenn die Elektronendichte im Ring in der Summe verringert ist.

Gegenstand der Erfindung ist ein Verfahren zur oxidativen elektrochemischen Aminierung von
a) 1-Nitronaphthalin,1-Cyanonaphthalin, 1-Alkoxy- bzw. 1- Aryloxycarbonylnaphthalin in 5-Position, bzw.
   a) Naphthalin in 1- und 5-Position
      an Bor-dotierten Diamantanoden.

Bevorzugt ist die Aminierung von 1-Nitronaphthalin in 5-Position, bzw. von Naphthalin in 1- und 5-Position.

Als Aminierungsreagenzien kommen vor allem Pyridin und seine substituierten sowie annelierten Abkömmlinge wie Picoline (2-, 3- und 4-Picolin), Lutidine (2,3-, 2,4-, 2,5-, 2,6-, 3,4- und 3,5-Lutidin) und Collidine (2,3,4-, 2,3,5-, 2,3,6-, 2,4,5-, 2,4,6- und 3,4,5-Collidin), gemischtalkylsubstituierte Pyridine, wie beispielsweise 5-Ethyllutidin und dessen Isomere, sowie Chinolin und Isochinolin in Frage.

In einer bevorzugten Ausführungsform werden Pyridin, ein oder mehrere Picolin-Isomere, ein oder mehrere Lutidin-Isomere, ein oder mehrere Collidin-Isomere, ein oder mehrere gemischtalkylsubstituierte Pyridin-Isomere, Chinolin, Isochinolin oder Mischungen dieser Verbindungen, bevorzugt 5-Ethyllutidin und/oder ein oder mehrere dessen Isomere, als Aminierungsreagenzien verwendet.

Gegenstand der Erfindung ist ein Verfahren zur elektrochemischen Aminierung von
b) 1-Nitronaphthalin,1-Cyanonaphthalin, 1-Alkoxy- bzw. 1-Aryloxycarbonylnaphthalin in 5-Position, bzw.
c) Naphthalin in 1- und 5-Position
an Bor-dotierten Diamantanoden unter Verwendung von Pyridin, einem oder mehreren Picolin-Isomeren, einem oder mehreren Lutidin-Isomeren, einem oder mehreren Collidin-Isomeren, Chinolin, Isochinolin oder Mischungen dieser Verbindungen als Aminierungsreagenzien.

Das bevorzugte Aminierungsreagenz ist Pyridin.

Für die anschließende Reaktion zur Freisetzung der primären Aminogruppe(n) bieten sich prinzipiell eine Reihe von Protonen tragenden Nucleophilen an, genannt seien Wasser, Hydroxid, Hydroperoxid, Ammoniak, Amid, Hydrazin, Hydrazid, Hydroxylamin, primäre oder sekundäre Amine der Formeln NH₂R¹ bzw. NHR¹R² -wobei R¹ und R² für lineare oder verzweigte, gesättigte oder ungesättigte, aliphatische, araliphatische, cycloaliphatische oder aromatische Reste mit 1 bis 10 Kohlenstoffatomen stehen, die gegebenenfalls Heteroatome aus der Reihe Sauerstoff, Schwefel, Stickstoff enthalten, oder im Falle von NHR¹R² untereinander gesättigte oder ungesättigte Cyclen mit 1 bis 6 Kohlenstoffatomen bilden, die gegebenenfalls Heteroatome aus der Reihe Sauerstoff, Schwefel, Stickstoff enthalten.

Bevorzugt sind Hydroxid, Ammoniak, Hydrazin, Hydroxylamin und Piperidin, besonders bevorzugt ist Piperidin.

Gegenstand der Erfindung ist auch ein Verfahren zur Herstellung von 1-Amino-5-nitronaphthalin, 1-Amino-5-Cyanonaphthalin, 1-Amino-5-Alkoxy- bzw. 1-Amino-5- Aryloxy-carbonylnaphthalin durch oxidative elektrochemische Aminierung von 1-Nitronaphthalin, 1-Cyanonaphthalin, 1-Alkoxycarbonylnaphthalin bzw. 1-Aryloxycarbonylnaphthalin in 5-Position an Bor-dotierten Diamantanoden und anschließender Aminfreisetzung aus dem primären Aminierungsprodukt. Bevorzugt ist die Herstellung von 1-Amino-5-nitronaphthalin aus 1-Nitronaphthalin nach diesem Verfahren, sowie auf diesem Wege hergestelltes 1-Amino-5-nitronaphthalin selbst.

Gegenstand der Erfindung ist auch ein Verfahren zur Herstellung von 1,5-Diaminonaphthalin durch oxidative elektrochemische Aminierung von Naphthalin in 1- und 5-Position an Bor-dotierten Diamantanoden und anschließender Aminfreisetzung aus dem primären Aminierungsprodukt. Ebenso sind weitere Gegenstände die beiden zuletzt beschriebenen Verfahren, wobei zur elektrochemischen Aminierung Pyridin und zur Aminfreisetzung aus dem primären Aminierungsprodukt Piperidin verwendet werden.

Durch die direkte elektrochemische Aminierung von 1-Nitronaphthalin in der 5-Position sowie der doppelten Aminierung von Naphthalin in 1,5-Position wird der klassische mehrstufige Syntheseprozess zum Diamin **2** drastisch verkürzt. Weiterhin können Ausbeuteverluste, die im klassischen Prozess bei der 1,5-Funktionalisierung von Naphthalin durch fehlende Regioselektivität und/oder Bildung von tri- und höhersubstituierten Zwischenprodukten auftreten, vermieden werden.

Die elektrochemische Aminierung von elektronenreichen aromatischen Systemen unter Verwendung von Graphit-Filz als Elektrodenmaterial wurde von Morofuji et al. 2013 publiziert (T. Morofuji, A. Shimizu, J.-I. Yoshida, J. Am. Chem. Soc. 2013, 135, 5000-5003). Im Gegensatz hierzu erfolgt bei der vorliegenden Erfindung die elektrochemische Aminierung elektronenarmer, d.h. nicht aktivierter bzw. sogar deaktivierter, aromatischer Verbindungen an Anoden aus bordotiertem Diamant (BDD). Eine Umsetzung der elektronenarmen Ausgangssubstanzen an anderen Anodenmaterialien (Graphit, Glaskohlenstoff, Platin) ist nicht möglich.

In der Literatur ist zudem die elektrochemische Aminierung von Anisol in Schwefelsäure/Acetonitril unter Verwendung von Ti(IV)/Ti(III) als Redoxmediator und Hydroxylamin als Stickstoffquelle beschrieben (Y. A. Lisitsin, L. V. Grigor'eva, Russ. J. Gen. Chem. 2008, 78, 1009-1010, Y. A. Lisitsyn, L. V. Grigor'eva, Russ. J. Electrochem. 2009, 45, 132-138, Y. A. Lisitsyn, A. V. Sukhov, Russ. J. Electrochem. 2011, 47, 1180-1185, Y. A. Lisitsyn, A. V. Sukhov, Russ. J. Phys. Chem. 2012, 86, 1033-1034, Y. A. Lisitsyn, A. V. Sukhov, Russ. J. Electrochem. 2013, 49, 91-95, Y. A. Lisitsyn, A. V. Sukhov, Russ. J. Gen. Chem. 2013, 83, 1457-1458.)

Ebenso ist in der Literatur die elektrochemische Synthese von Nitroanilinen aus den entsprechenden aromatischen Nitroverbindungen bekannt. In einem der Oxidation vorgelagertem Reaktionsschritt findet hier ein nukleophiler Angriff eines geeigneten Stickstoff-Nukleophils auf einen elektronenarmen Nitroaromaten statt. Oxidation des intermediär entstehenden Meisenheimer-Komplexes liefert schließlich die substituierte aromatische Nitroverbindung. 1-Nitronaphthalin wird nach dieser Methode in 2-Position aminiert, 1,3-Dinitronaphthalin in 4-Position. So ist nur eine Funktionalisierung von Ring A möglich (H. Cruz, I. Gallardo, G. Guirado, Green Chem. 2011, 13, 2531-2542, I. Gallardo, G. Guirado, J. Marquet, Eur. J. Org. Chem. 2002, 2002, 251-259).

Die elektrochemische Aminierung von 1-Nitronaphthalin **6** gemäß der vorliegenden Erfindung verläuft über die intermediäre Bildung eines Radikal-Kations an der BDD-Anode welches von Pyridin bzw. seinen o.g. Derivaten als Nukleophil abgefangen wird (in Schema 2 beispielhaft mit Pyridin dargestellt). Als Lösungsmittel kann beispielsweise Acetonitril verwendet werden. Da der A-Ring von 1-Nitronaphthalin stärker deaktiviert ist als der B-Ring erfolgt der Angriff von Pyridin bevorzugt in 5-Position (Schema 2). In dieser Position befinden sich die Substituenten zudem in maximaler Entfernung zueinander. Aminfreisetzung aus dem primären Naphthylpyridiniumsystem **7** liefert schließlich 1-Amino-5-nitronaphthalin **8** (Schema 2), welches katalytisch zum gewünschten Diamin **2** hydriert werden kann.

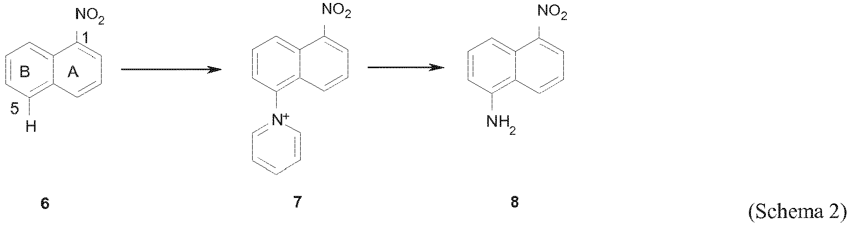

Weiterhin wird das Problem der selektiven Einführung von zwei Aminofunktionen in 1,5-Position von Naphthalin durch die doppelte elektrochemische Aminierung von Naphthalin gelöst. Das intermediär an der BDD-Anode gebildete Radikal-Kation wird von Pyridin bzw. seinen o.g. Derivaten als Nukleophil abgefangen (in Schema 3 beispielhaft mit Pyridin dargestellt). Als Lösungsmittel kann beispielsweise Acetonitril verwendet werden. Da bei der zunächst gebildeten einfachen 1-Naphthylpyridinium-Zwischenstufe bereits eine positive Ladung in 1-Position vorhanden ist, greift das zweite Pyridinmolekül bzw. sein o.g. Derivat aus elektrostatischen Gründen in 5-Position an (in Schema 3 beispielhaft mit Pyridin dargestellt). Aminolyse der 1,5-Naphthyldipyridinium-Verbindung **7** mit Piperidin liefert das gewünschte Diamin **2** (Schema 3).

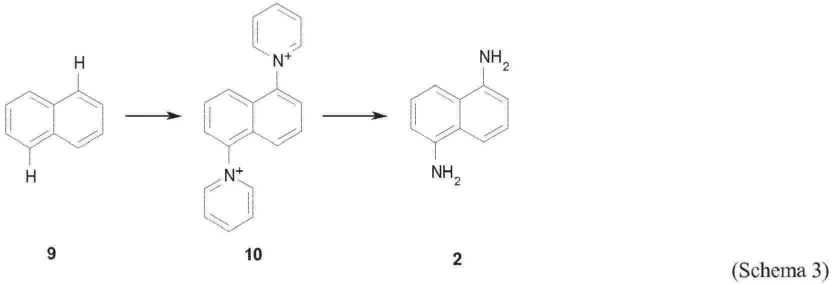

Durch diese direkte Methode der Aminierung wird eine kostengünstige und umweltschonende Alternative zu der bisher bestehenden mehrstufigen Syntheseroute eröffnet.

### Beispiele

Figur 1: 1,5-Naphthalindiisocyanat (**1**) und 1,5-Diaminonaphthalin (**2**).
Figur 2: Geteilte Teflonzellen im Screeningblock (links), geteilte H-Zelle mit BDD-Elektroden und Magnetrührkernen (rechts); Größe der Elektroden: jeweils 10 x 70 mm; Trennung von Anoden- und Kathodenraum erfolgt jeweils über einen porösen Separator aus gesintertem Glas der Porosität 4 mit 10 mm Durchmesser; Lösungsmittelvolumen: jeweils 6 mL; Elektrodenabstand: 250 mm (Teflonzelle), 350 mm (H-Zelle).
Figur 3: Kristallstruktur von l-Amino-5-nitronaphthalin (**8**).

### Chromatographie:

Die präparativen flüssigkeitschromatographischen Trennungen via "Flashchromatographie" wurden mit 1.6 bar Maximaldruck an Kieselgel 60 M (0.040-0.063 mm) der Firma *Macherey-Nagel GmbH & Co,* Düren durchgeführt. Die Trennungen ohne Druckbeaufschlagung wurden an Kieselgel Geduran Si 60 (0.063-0.200 mm) der Firma *Merck KGaA,* Darmstadt durchgeführt. Die als Eluenzien verwendeten Lösungsmittel (Essigsäureethylester (technisch), Cyclohexan (technisch)) wurden zuvor destillativ am Rotationsverdampfer gereinigt.

Zur Dünnschichtchromatographie (DC) wurden PSC-Fertigplatten Kieselgel 60 F254 der Firma *Merck KGaA,* Darmstadt verwendet. Die Rf-Werte (Retentionsfaktor) sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5,6 g Molybdatophosphorsäure, 2,2 g Cer(IV)-sulfat-Tetrahydrat und 13,3g konzentrierte Schwefelsäure auf 200 mL Wasser.

### Gaschromatographie (GC/GCMS):

Die gaschromatographischen Untersuchungen (GC) von Produktgemischen und Reinsubstanzen erfolgte mit Hilfe des Gaschromatographen GC-2010 der Firma *Shimadzu,* Japan. Es wird an einer Quarzkapillarsäule HP-5 der Firma *Agilent Technologies,* USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma *Shimadzu,* Japan aufgenommen. Es wird an einer Quarzkapillarsäule HP-1 der Firma *Agilent Technologies,* USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der Ionenquelle: 200 °C) gemessen.

### Massenspektrometrie:

Alle Elektrosprayionisation-Messungen (ESI+) wurden an einem QTof Ultima 3 der Firma *Waters Micromasses,* Milford, Massachusetts durchgeführt.

### NMR-Spektroskopie:

Die NMR-spektroskopischen Untersuchungen wurden an Multikernresonanzspektrometern des Typs AC 300 oder AV II 400 der Firma *Bruker,* Analytische Messtechnik, Karlsruhe, durchgeführt. Als Lösungsmittel wurde d₆-DMSO verwendet. Die ¹H- und ¹³C-Spektren wurden gemäß dem Restgehalt an nicht deuteriertem Lösungsmittel nach der *NMR Solvent Data Chart* der Fa. *Cambridge Isotopes Laboratories,* USA, kalibriert. Die Zuordnung der ¹H- und ¹³C-Signale erfolgte teilweise mit Hilfe von H,H-COSY (correlation spectroscopy), H,C-HSQC (heteronuclear single quantum coherence spectroscopy) und H,C-HMBC-Spektren (heteronuclear multiple bond correlation spectroscopy). Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten *J* wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Einkristallstrukturanalysen:

Die Einkristallstrukturanalysen wurden im Institut für Organische Chemie der Johannes Gutenberg-Universität Mainz an einem Gerät des Typs IPDS 2T der Firma *STOE & Cie GmbH,* Darmstadt durchgeführt.

### Allgemeine Arbeitsvorschrift zur elektrochemischen Aminierung:

Die elektrochemische Reaktion wurde in einer geteilten Teflonzelle bzw. H-Zelle durchgeführt (Figur 2). Als Anodenmaterial wurde bor-dotierter Diamant verwendet. Als Kathodenmaterial kam Platin zum Einsatz. In den Anodenraum wurde eine Lösung bestehend aus der jeweiligen aromatischen Verbindung (0,1 mol x L⁻¹) und Pyridin (2,4 mol x L⁻¹, trocken) in 0,2 molarer Bu₄NBF₄/Acetonitril-Mischung (5 mL, trocken) gegeben. In den Kathodenraum wurde eine Lösung aus Trifluormethansulfonsäure (0,4 mL) in 0,2 molarer Bu₄NBF₄/Acetonitril-Mischung (5 mL, trocken) gegeben. Die Elektrolysen wurden galvanostatisch bei 60 °C durchgeführt.

Nachdem die jeweiligen Ladungsmengen erreicht worden sind, wurde die Reaktionslösung in einen 100 mL Rundkolben überführt und Acetonitril unter vermindertem Druck entfernt. Anschließend wurden 10 mL Acetonitril hinzugegeben sowie 1 mL Piperidin und die Lösung bei 80 °C für 12 h unter Rückfluss erhitzt. Die Reaktionsmischung wurde mittels GC, DC und GC/MS auf die Aminierungsprodukte überprüft.

Schema 2 zeigt die direkte elektrochemische Aminierung von 1-Nitronaphthalin zu 1-Amino-5-nitronaphthalin an bor-dotierten Diamant Anoden. Beispielhaft wurden folgende Mengen, Materialen und Verfahrensparameter gewählt: 0.5 mmol 1-Nitronaphthalin; 12 mmol Pyridin; 0.2 M Bu₄NBF₄/Acetonitril; Lösungsmittel: Acetonitril; Anode: BDD (1.5 cm²); Kathode: Platin (1.5 cm²); Temperatur: 60 °C; Ladungsmenge: 144 C; Stromdichte: j = 10 mA cm⁻². Aminfreisetzung: Piperidin; Lösungsmittel: Acetonitril.

Schema 3 zeigt die direkte elektrochemische Aminierung von Naphthalin zu 1,5-Diaminonaphthalin an bor-dotierten Diamant Anoden. Beispielhaft wurden folgende Mengen, Materialen und Verfahrensparameter gewählt: 0.5 mmol 1-Nitronaphthalin; 12 mmol Pyridin; 0.2 M Bu₄NBF₄/Acetonitril; Lösungsmittel: Acetonitril; Anode: BDD (1.5 cm²); Kathode: Platin (1.5 cm2); Temperatur: 60 °C; Ladungsmenge: 288 C; Stromdichte: j = 10 mA cm⁻². Aminfreisetzung: Piperidin; Lösungsmittel: Acetonitril; Temperatur: 80 °C; Reaktionszeit: 12 Stunden.

### Beispiel 1 (erfindungsgemäß): Herstellung von 1-Amino-5-nitronaphthalin (8).

Gemäß obiger Arbeitsvorschrift wurden 0,09 g (0,53 mmol, 0,04 Äquivalente) 1-Nitronaphthalin, 0,30 g (0,91 mmol, 0,07 Äquivalente) Tetrabutylammoniumtetrafluoroborat, 1 mL (0,98 g, 12,41 mmol, 1,0 Äquivalente) Pyridin in 5 mL trockenem Acetonitril gelöst und in den Anodenraum gegeben. In den Kathodenraum wurde eine Lösung aus 0,3 g (0,91 mmol) Tetrabutylammoniumtetrafluoroborat und 0,4 mL Trifluormethansulfonsäure in 5 mL trockenem Acetonitril gegeben. Die Elektrolyse wurde in einer geteilten Teflonzelle durchgeführt.
Anode: BDD; Elektrodenfläche: 1,5 cm².
Kathode: Platin; Elektrodenfläche: 1,5 cm².
Ladungsmenge: 144 C.
Stromdichte: j = 10 mA cm⁻².
Temperatur: 60 °C.

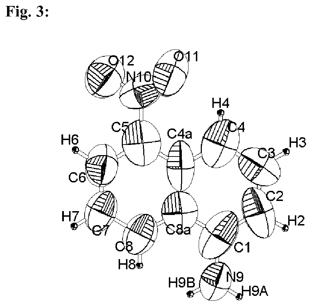

Nach Ablauf der Elektrolysezeit wurde das Lösungsmittel unter vermindertem Druck entfernt. Anschließend wurden 10 mL Acetonitril hinzugegeben sowie 1 mL (0,86 g, 10,00 mmol, 0,8 Äquivalente) Piperidin. Die Reaktionslösung wurde bei 80 °C für 12 h unter Rückfluss erhitzt. Das Lösungsmittel wurde unter vermindertem Druck entfernt und der Rückstand säulenchromatographisch unter Verwendung eines Trennsystems der *Fa. Büchi-Labortechnik GmbH* an Kieselgel aufgereinigt. Es wurde eine 150 mm lange Trennsäule mit 40 mm Durchmesser verwendet und bei 10 bar Maximaldruck sowie 50 mL x min⁻¹ Flussgeschwindigkeit gearbeitet. Hierbei wurde folgender Lösungsmittelgradient verwendet: zunächst Cyclohexan/Ethylacetat 95:5 anschließend wurde der Lösungsmittelgradient innerhalb der ersten 40 min auf Cyclohexan/Ethylacetat 3:1 (R_{F} = 0,16) erhöht. Es sind 19,6 mg (0,1 mmol, 21%) eines rötlichen Feststoffes erhalten worden.
¹H-NMR (400 MHz, DMSO): δ (ppm) = 6,18 (s, 2 H, NH₂), 6,83 (dd, ³*J* = 7,2 Hz, ⁴*J* = 1,3 Hz, 1 H, H-2), 7,35-7,56 (m, 3 H, H-3,4,7), 8,14 (d, ³*J* = 7,5 Hz, 1 H, H-8), 8,49 (d, ³*J* = 8,5 Hz, 1 H, H-6).
¹³C-NMR (101 MHz, DMSO): δ (ppm) = 108,4 (C-4), 108,8 (C-2), 121,8, 123.37 (C-8), 125,4 (C-4a), 128,2 (C-6), 130,6, 146,0 (C-1), 146,6 (C-5).
GC (Methode *hart,* HP-5): t_{R} = 12,78 min (Retentionszeit).
HRMS (High Resolution Mass Spectromertry) berechnet für C₁₀H₉N₂O₂: 189,0664; gefunden: 189,0662.
Kristallstrukturanalyse (Kristallstruktur: Figur 3):

Der Einkristall wurde durch Kristallisation von 20 mg **8** aus *n*-Heptan/Methanol erhalten.

| | |
|---|---|
| Summenformel | C₁₀H₈N₂O₂ |
| Molekulargewicht | 188,2 g mol⁻¹ |
| Temperatur | -80 °C |
| Strahlung | Cu-K_{α} |
| Kristallsystem, Raumgruppe | orthorhombisch, P na2₁ |
| Gitterkonstanten | a = 12,935(2)Å |
| | b = 16,996(4)Å |
| | c = 3,9407(7)Å |
| Zellvolumen | V = 866,3(3)Å³ |
| Z, berechnete Dichte | 4, 1,443 g cm⁻³ |
| Absorptionskoeffizient | 0,86 mm⁻¹ |
| F(000) | 392 |
| Kristallgröße | 0,01 x 0,01 x 0,6 mm³ braune Nadel |
| Θ-Bereich der Datensammlung | 2,6-67,8° |
| Reflexbereich | -15 ≤ h ≤ 12, -20 ≤ k ≤ 16, -4 ≤ 1 ≤ 4 |
| Gemessene/unabh. Reflexe | 1501 (Rᵢₙₜ = 0,3445) |
| Diskrepanzfaktor | wR2 = 0,4938 (R1 = 0,1574 für beobachtete Reflexe 0,4437 für alle Reflexe) |
| Fitgüte | S = 0,808 |

Die Strukturdaten der Verbindung sind unter der Bezeichnung SHE1332 im Organischen Institut der Universität Mainz hinterlegt (Dr. D. Schollmeyer, Röntgenstrukturanalyse, Duesbergweg 10-14, 55128 Mainz).

### Beispiel 2 (erfindungsgemäß): Herstellung von 1,5-Diaminonaphthalin (2).

Gemäß der allgemeinen Arbeitsvorschrift wurden 0,064 g (0,50 mmol, 0,04 Äquivalente) Naphthalin, 0,30 g (0,91 mmol, 0,07 Äquivalente) Tetrabutylammoniumtetrafluoroborat, 1 mL (0,98 g, 12,41 mmol, 1,0 Äquivalente) Pyridin in 5 mL trockenem Acetonitril gelöst und in den Anodenraum gegeben. In den Kathodenraum wurde eine Lösung aus 0,3 g (0,91 mmol) Tetrabutylammoniumtetrafluoroborat und 0,4 mL Trifluormethansulfonsäure in 5 mL trockenem Acetonitril gegeben. Die Elektrolyse wurde in einer geteilten H-Zelle durchgeführt.
Anode: BDD; Elektrodenfläche: 1,5 cm².
Kathode: Platin; Elektrodenfläche: 1,5 cm².
Ladungsmenge: 288 C.
Stromdichte: j = 10 mA cm⁻².
Temperatur: 60 °C.

Nach Ablauf der Elektrolysezeit wurde das Lösungsmittel unter vermindertem Druck entfernt. Anschließend wurden 10 mL Acetonitril hinzugegeben sowie 1 mL (0,86 g, 10,00 mmol, 0,8 Äquivalente) Piperidin. Die Reaktionslösung wurde bei 80 C für 12 h unter Rückfluss erhitzt. Das Lösungsmittel wurde nach vollendeter Reaktionszeit unter vermindertem Druck entfernt und der Rückstand säulenchromatographisch an Kieselgel aufgereinigt (Cyclohexan/Ethylacetat 2:1, R_{F} = 0.39). Es wurden 0,01 g (0,06 mmol, 10%) eines braunen Feststoffes erhalten.
¹H-NMR (400 MHz, DMSO): δ (ppm) = 5,41 (s, 4 H, NH₂), 6,60 (dd, ³*J* = 7,2 Hz, ⁴*J* = 1,1 Hz, 2 H, H-2), 7,05 (dd, ³*J* = 8,4 Hz, 7,2 Hz, 2 H, H-3) 7,17-7,24 (m, 2 H, H-4).
¹³C-NMR (101 MHz, DMSO): δ (ppm) = 107,4 (C-2), 110,0 (C-4), 123.8 (C-4a), 124.3 (C-3), 144,5 (C-1).
GC (Methode *hart,* HP-5): t_{R} = 11,89 min.
HRMS berechnet für C₁₀H₁₁N₂: 159,0922; gefunden: 159,0920.
MS (EI, 70 eV): m/z(%): 158 (100) [M]˙⁺

## Patentansprüche

1. Verfahren zur oxidativen elektrochemischen Aminierung von
a) 1-Nitronaphthalin, 1-Cyanonaphthalin, 1-Alkoxycarbonylnaphthalin oder 1-Aryloxycarbonylnaphthalin in 5-Position, bzw.
b) Naphthalin in 1- und 5-Position
an Bor-dotierten Diamantanoden.

2. Verfahren gemäß Anspruch 1 unter Verwendung von Pyridin, einem oder mehreren Picolin-Isomeren, einem oder mehreren Lutidin-Isomeren, einem oder mehreren Collidin-Isomeren, Chinolin, Isochinolin oder Mischungen dieser Verbindungen als Aminierungsreagenzien.

3. Verfahren gemäß Anspruch 1, wobei Pyridin als Aminierungsreagenz verwendet wird.

4. Verfahren gemäß Anspruch 1 unter Verwendung von Pyridin, einem oder mehreren Picolin-Isomeren, einem oder mehreren Lutidin-Isomeren, einem oder mehreren Collidin-Isomeren, einem oder mehreren gemischt-alkylsubstituierten Pyridin-Isomerem, Chinolin, Isochinolin oder Mischungen dieser Verbindungen, bevorzugt von 5-Ethyllutidin und/oder einem oder mehrerer dessen Isomere, als Aminierungsreagenzien.

5. Verfahren gemäß einem der Ansprüche 1 bis 4 mit anschließender Aminfreisetzung aus dem primären Aminierungsprodukt..

6. Verfahren gemäß Anspruch 5, wobei das Verfahren ein Verfahren zur Herstellung von 1,5-Diaminonaphthalin ist.

7. Verfahren gemäß Anspruch 5 oder 6, wobei für die Aminfreisetzung Hydroxid, Ammoniak, Hydrazin, Hydroxylamin oder Piperidin, bevorzugt Piperidin verwendet wird.

## Claims

1. Process for the oxidative electrochemical amination of
a) 1-nitronaphthalene, 1-cyanonaphthalene, 1-alkoxycarbonylnaphthalene or 1-aryloxycarbonylnaphthalene in the 5 position, or
b) naphthalene in the 1 and 5 positions
at boron-doped diamond anodes.

2. Process according to Claim 1 using pyridine, one or more picoline isomers, one or more lutidine isomers, one or more collidine isomers, quinoline, isoquinoline or mixtures of these compounds as amination reagents.

3. Process according to Claim 1, wherein pyridine is used as amination reagent.

4. Process according to Claim 1 using pyridine, one or more picoline isomers, one or more lutidine isomers, one or more collidine isomers, one or more pyridine isomers having mixed alkyl substituents, quinoline, isoquinoline or mixtures of these compounds, preferably 5-ethyllutidine and/or one or more isomers thereof, as amination reagents.

5. Process according to any of Claims 1 to 4, comprising subsequent setting-free of the amine from the primary amination product.

6. Process according to Claim 5, wherein the process is a process for preparing 1,5-diaminonaphthalene.

7. Process according to Claims 5 or 6, wherein hydroxide, ammonia, hydrazine, hydroxylamine or piperidine, preferably piperidine, is used for the setting-free of the amine.

## Revendications

1. Procédé d'amination électrochimique oxydative de
a) 1-nitronaphtaline, 1-cyanonaphtaline, 1-alcoxycarbonylnaphtaline ou 1-aryloxycarbonylnaphtaline en position 5, ou
b) naphtaline en position 1 et 5,
sur des anodes en diamant dopées au bore.

2. Procédé selon la revendication, utilisant de la pyridine, un ou plusieurs isomères de picoline, un ou plusieurs isomères de lutidine, un ou plusieurs isomères de collidine, de la quinoline, de l'isoquinoline ou des mélanges de ces composés en tant que réactifs d'amination.

3. Procédé selon la revendication 1, dans lequel la pyridine est utilisée en tant que réactif d' amination.

4. Procédé selon la revendication 1, utilisant de la pyridine, un ou plusieurs isomères de picoline, un ou plusieurs isomères de lutidine, un ou plusieurs isomères de collidine, un ou plusieurs isomères de pyridine à substitution alkyle mixte, de la quinoline, de l'isoquinoline ou des mélanges de ces composés, de préférence de la 5-éthyllutidine et/ou un ou plusieurs de ses isomères, en tant que réactifs d'amination.

5. Procédé selon l'une quelconque des revendications 1 à 4, comprenant une libération ultérieure de l'amine du produit d'amination primaire.

6. Procédé selon la revendication 5, dans lequel le procédé est un procédé de fabrication de 1,5-diaminonaphtaline.

7. Procédé selon la revendication 5 ou 6, dans lequel un hydroxyde, de l'ammoniac, de l'hydrazine, de l'hydroxylamine ou de la pipéridine, de préférence de la pipéridine, est utilisé pour la libération de l'amine.
